# EUROPEAN PATENT APPLICATION

(11) **EP 1 696 039 A2**
(43) Date of publication of application: **30.08.2006**
(21) Application number: 06110508.6
(22) Date of filing: 28.02.2006
(51) Int. Cl.: C12Q 1/68, G01N 33/569, C12N 15/11

(54) **Lactobacillus specific probes**

(30) Priority: 28.02.2005 EP 05075486
(71) Applicant: N.V. Nutricia, 2712 HM Zoetermeer (NL)
(72) Inventor: Knol, Jan, 6708 SM Wageningen (NL); Haarman, Monique, 6702 BW Wageningen (NL)
(74) Representative: van Westenbrugge, Andries

(57) **Abstract**

Provided herein are sequence primers and probes for the detection of *Lactobacillus* species as well as diagnostic kits thereof.

## Description

### TECHNICAL FIELD OF THE INVENTION

The present invention relates to probes and primers for quantification of *Lactobacillus* species.

### BACKGROUND OF THE INVENTION

Generally, the intestinal flora of human milk-fed infants is primarily composed of the genus *Bifidobacterium* and *Lactobacillus.* The genera *Bifidobacterium* and *Lactobacillus* are considered to be important in maintaining a well-balanced intestinal microbiota and it has been postulated that they have several health-promoting effects.

The approach up to now was to promote *Lactobacillus* in general, i.e. on the genus level. The genus *Lactobacillus* consists of many different species, which differ in metabolism, metabolites, enzyme activities, oligo- and polysaccharide utilisation, cell wall composition, and interaction with the host's immune system. It therefore can be expected that not every species of the genus *Lactobacillus* has the same functional effect on the infant. Examples of different *Lactobacillus* species are *L. rhamnosus, L. casei, L. paracasei, L. reuteri, L. fermentum, L. plantarum, L. acidophilus,* and *L. delbruecki,.* For example, species producing large amounts of D-lactate, such as *L. delbrueckii,* may cause acidosis in young infants. These results indicate that the stimulation of the genus *Lactobacillus* per se in the babies' colon may not be sufficient. It is therefore the aim to achieve a *Lactobacillus* flora in formula-fed infants that is reminiscent to the *Lactobacillus* flora of human milk fed babies on a species level.

So far, quantification of *Lactobacillus* on species level in faecal samples or intestinal biopsies has not been adequately performed. Plating, followed by colony determination and conventional PCR techniques only result in qualitative or semi-quantitative data. Fluorescence *in situ* hybridisation is not suitable, because of the low concentration of *Lactobacillus* species and the available FISH probes are not sufficiently specific.

Therefore, there is a need for a method to quantify *Lactobacillus* species distribution in the gastrointestinal tract and/or faeces of infants.

EP0674650 relates to nucleic acid sequences which preferentially bind to the rRNA or rDNA of microorganisms which cause the spoilage of beer. The beer spoilage microorganisms are predominantly of the genera *Lactobacillus* and *Pediococcus.* The nucleic acids may be used as probes in assays to detect the presence of these microorganisms. Kits containing two or more probes are also described.

### SUMMARY OF THE INVENTION

In order to quantitatively determine the *Lactobacillus* species distribution in faeces real-time PCR (5'nuclease assay) has to be used, for which species specific probes and primers are provided.

A method of species-specifically detecting and quantitatively assaying species of the genus *Lactobacillus* found in humans, particularly human infants, as well as a diagnostic kit for the detection and quantification *of Lactobacillus* species is provided.

### DETAILED DESCRIPTION OF THE INVENTION

### Probe development and diagnostic kit

A method of species-specifically detecting and quantitatively assaying species of the genus *Lactobacillus* found in human faeces and/or intestine, particularly human infants, as well as a diagnostic kit for the detection and quantification *of Lactobacillus* species is provided.

Also provided herein is a method for quantifying *Lactobacillus* species, especially those found in humans, *i.e. L. acidophilus, L. casei, L. delbrueckii, L. fermentum. L. paracasei, L. plantarusm, L. reuteri* and *L rhamnosus* using species specific oligonucleotide primes and probes.

These primers and probes can be used to identify species of *Lactobacillus* and

*Lactobacillus-like* species via FISH, PCR, DGGE, TGGE, dot blot hybridisation and real time PCR methods. All these techniques have in common that it involves a hybridisation step with nucleotides. Preferably real time PCR is used to quantify *Lactobacillus* species.

Each of the sequences described below may have additional bases bonded to the 5'- or 3'-terminal thereof as long as it functions as a probe.

These oligonucleotides can be prepared by conventional means for chemical synthesis, for example by an automated DNA synthesiser. DNA fragments containing the above-mentioned sequences can be prepared by enzymatic cleavage of genes from the corresponding *Lactobacillus* species.

For the purpose of the present invention, the development of primers and probes specific to the *Lactobacillus* species for use in the 5'nuclease assay was as follows:
Duplex 5' nuclease assays were developed *for L. acidophilus, L. casei, L. delbrueckii, L. fermentum, L. paracasei, L. plantarum, L. reuteri* and *L rhamnosus* as a proportion of the total cells of the genus *Lactobacillus.* We developed the 5' nuclease assays on the intergenic spacer of 16S-23S rDNA instead of the 16S rDNA gene, which is normally used for the phylogenetic analyses and specific detection of bacteria. The choice for the intergenic spacer greatly depended on the fact that contamination and sensitivity issues were described for Real-Time PCR when 16S rDNA was used. Furthermore, a large similarity between the 16S rDNA sequences of the different *Lactobacillus* species was shown (Leblond-Bourget et. al. 1996), which made it almost impossible to develop primer and probe sets specific for the different *Lactobacillus* species. Surprisingly, these problems could be avoided by using the intergenic spacer region.

For the development of primers and probes the different sequences of the 16S-23S intergenic spacer region of the different *Lactobacillus* species *(L. acidophilus* [AB102855 (3), AF182726 (5), U32971 (7)]^{a}, *L. amylovorus* [AF182732 (5)], *L. animalis* [AY526616, AY526614], *L. brevis* [AB102858 (3), AF405353 (2)], *L. bulgaricus* [Z75475], *L. casei* [AB102854 (3), AF405352 (2), AF182729 (5), AF121200 (6)], *L. collinoides* [AB117957, AB117955], *L. crispatus* [AF182719 (5), AF074857 (6)], *L. curvatus* [AF074858 (6), U97135 (1), U97129 (1)], *L. delbrueckii* [AB102856 (3), AB035485 (5), AB035484 (5), U32969 (7), U32968 (7), U32967 (7)], *L. farciminis* [AF500491 (4), AF500490 (4)], *L. fermentum* [AF182720 (5)], *L. frumenti* [AJ616011], *L. gasseri* [AB102860 (3), AF182721 (5), AF074859 (6)], *L. graminis* [U97136 (1), U97130 (1)], *L. hamsteri, L. helveticus* [AF182728 (5)], *L. jensenii* [AB035486 (5), U32970 (7)], *L. johnsonii* [AF074860 (6)], *L. mindensis* [AJ616016], *L. panis* [AJ616012], *L. paracasei* [AB035487 (5), AF182724 (5), U32964 (7)], *L. paralimentarius* [AJ616014], *L. paraplantarum* [U97138 (1), U97132 (1)], *L. pentosus* [U97141 (1), U97140 (1), U97134 (1)], *L. plantarum* [AB102857 (3), AF405354 (2), AF182722 (5), U97139 (1), U97133 (1)], *L. sakei* [U97137 (1), U97131 (1)], *L. salivarius* [AB102859 (3), AB03488 (5), AF182725 (5)], *L. sharpeae* [AF074861 (6)], *L. reuteri* [AF182723 (5)], *L. rhamnosus* [AF182730 (5), AF121201 (6), U32966 (7)], *L. ruminis* [AF080103], *L. vaginalis* [AF182731], *L. zeae* [AF074862]^{a}) were retrieved from Genbank, EMBL and DDBJ databases. All retrieved sequences were aligned using DNASIS for Windows V2.5 (Hitachi Software Engineering Co., Ltd., Wembley, UK). (^{a} =accession codes, 1: Berthier et al. 1998. FEMS Microbiol Lett 161:97-106. 2: Dobson et al. 2002. Int J Syst Evol Microbiol 52:2003-10. 3: Massi et al. 2004. J Appl Microbiol 96:777-86. 4: Rachman et al. 2003. J Appl Microbiol 95:1207-16. 5: Song et al. 2000. FEMS Microbiol Lett 187:167-73. 6: Tannock et al.1999 Appl Environ Microbiol 65:4264-7. 7: Tilsala-Timisjarvi, A., and T. Alatossava. 1997. Int J Food Microbiol 35:49-56.)

The overall conserved regions of the sequences were used to design primers and probes for the *Lactobacillus* genus. To increase specificity and sensitivity, TaqMan Minor groove Binding (MGB) probes were used. Conserved regions in the sequences of the different kind of subspecies, which showed little homology with other species were used to design primers and probes for respectively *L. acidophilus, L. casei, L. delbrueckii, L. fermentum, L. paracasei, L. plantaurum, L. rhamnosus* and *L. reuteri.*

The primers and TaqMan MGB probes were designed with help of Primer Express 1.5a (Applied Biosystems, Nieuwerkerk a/d IJssel, NL). We applied the following criteria: The probe and primers should have a GC content of 30 to 80% and runs of more than 3 identically nucleotides (especially for guanidine (G)) should be avoided. The melting temperature (Tm) of the probe should be between 68 °C and 70 °C, whereas the primers should have a melting temperature 10 °C below the melting temperature of the probe. Furthermore, no G on the 5' end of the probe should be present and the strand with more cytosine (C) than G was selected. The last 5 nucleotides at the 3' end of the primers should have no more than two G and/or C bases. Finally, the amplicon length should be less than 150 base pairs. The designed primers and TaqMan MGB probes are shown in table 1 and were tested on specificity using the Basic Local Alignment Search Tool (BLAST).

The probe designed for the detection of the genus *Lactobacillus* consists of an oligonucleotide with the 5' reporter dye VIC™ (Applied Biosystems, NL) and the 3' quencher NFQ-MGB™ (Applied Biosystems, NL) and the probes for the different *Lactobacillus* species of oligonucleotides with the 5' reporter dye 6-carboxy-fluorescein (FAM™) and the 3' quencher NFQ-MGB™ (Applied Biosystems, NL). For determination of the total bacterial load a broad-range (universal) probe and primer set is used, which is described by Nadkarni, et al,2002, Microbiology 148:257-266. The universal probe consists of oligonucleotides with the 5' reporter dye 6-carboxy-fluorescein (FAM™) and the 3' quencher dye 6-carboxy-tetramethyl-rhodamine (TAMRA™). The designed probes are shown in table 1.

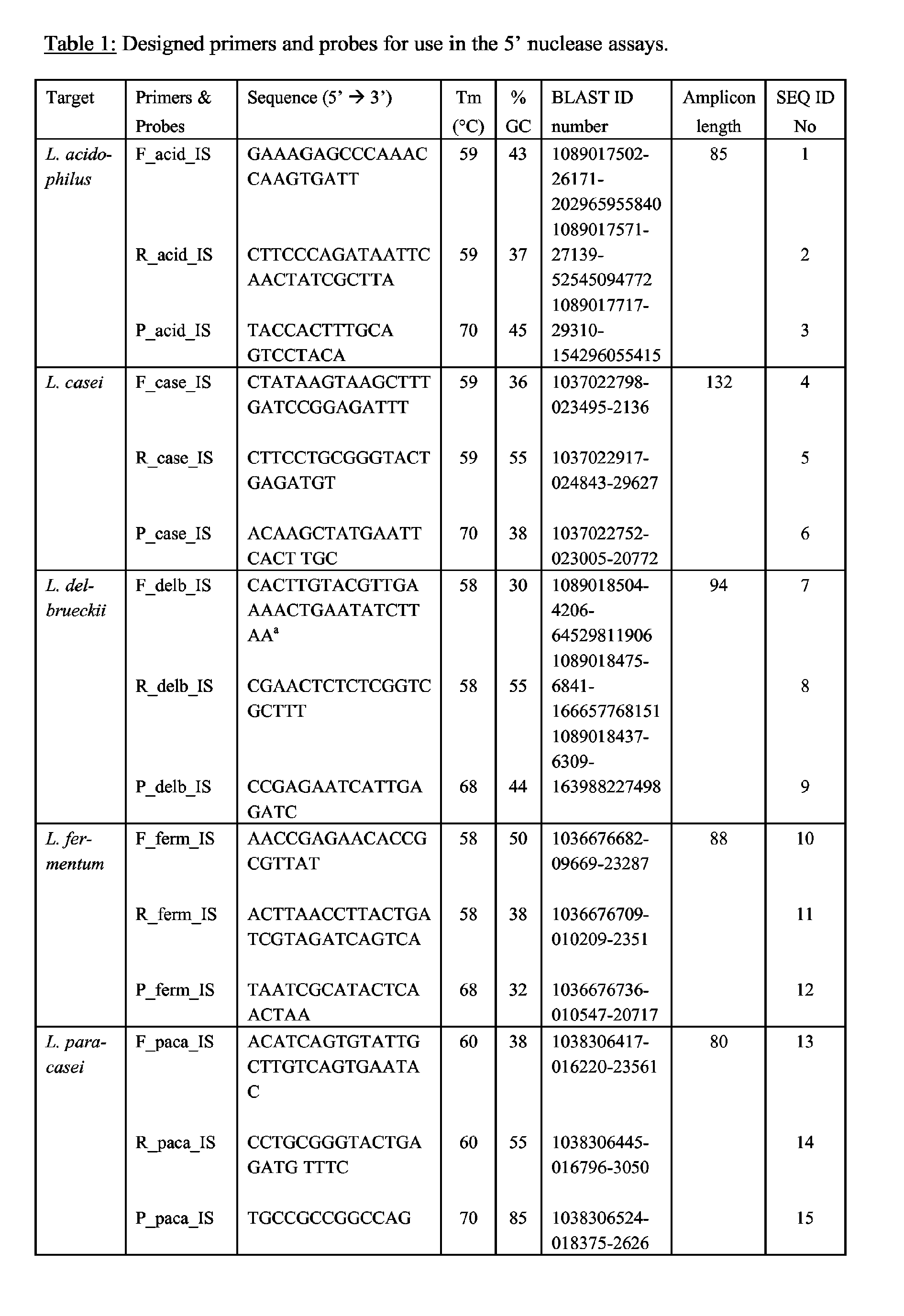

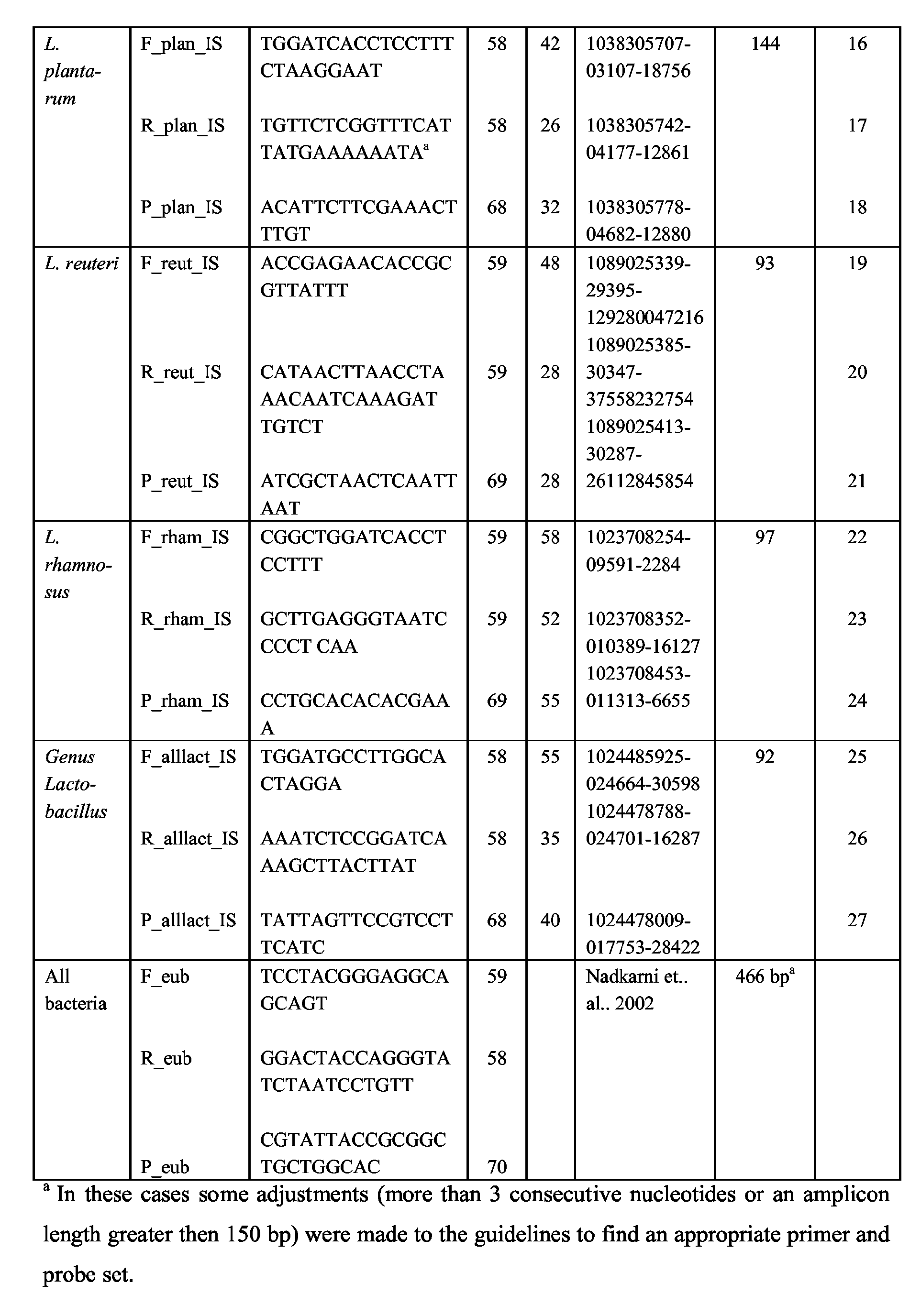

Labelled preparations are prepared by labelling the oligonucleotide with a detectable marker by conventional means. Labelling markers which may be used include radioisotopes, fluorescent substances, enzymes, biotin and haptens.

Hybridisation between the labelled preparation and a sample can be performed in accordance with known techniques, such as dot blot hybridisation and northern hybridisation. The hybrid which are formed can be confirmed through the detection of the labelled preparation by known means, for example, autoradiography using radioisotopes, enzyme-labelled antibody techniques using enzyme or biotin, and the like.

Further, of these oligonucleotides, the DNA fragments represented by SEQ ID selected from SEQ ID No 1, SEQ ID No 2, SEQ ID No 4, SEQ ID No 5, SEQ ID No 7, SEQ ID No 8, SEQ ID No 10, SEQ ID No 11, SEQ ID No 13, SEQ ID No 14, SEQ ID No 16, SEQ ID No 17, SEQ ID No 19, SEQ ID No 20, SEQ ID No 22, SEQ ID No 23, SEQ ID No 25, and SEQ ID No 26 respectively can be used as a primer in the PCR method for identification of species. More specifically, microbial cells to be identified are subjected to bacteriolysis, and any of the DNA fragments of SEQ ID selected from SEQ ID No 1, SEQ ID No 4, SEQ ID No 7, SEQ ID No 10, SEQ ID No 13, SEQ ID No 16, SEQ ID No 19, SEQ ID No 22, SEQ ID No 25, respectively and SEQ ID selected from SEQ ID No 2, SEQ ID No 5, SEQ ID No 8, SEQ ID No 11, SEQ ID No 14, SEQ ID No 17, SEQ ID No 20, SEQ ID No 23, SEQ ID No 26, respectively is added thereto as a primer, followed by treatment with a DNA polymerase. If DNA amplification is observed using electrophoresis, etc., this means that the cells possess a gene portion which corresponds to the DNA fragment used, i.e. the cells are identified to be of the same species as the origin of the DNA fragment primer.

Accordingly, there are provided oligonucleotides comprising SEQ ID selected from SEQ ID No 1, SEQ ID No 2, SEQ ID No 4, SEQ ID No 5, SEQ ID No 7, SEQ ID No 8, SEQ ID No 10, SEQ ID No 11, SEQ ID No 13, SEQ ID No 14, SEQ ID No 16, SEQ ID No 17, SEQ ID No 19, SEQ ID No 20, SEQ ID No 22, SEQ ID No 23, SEQ ID No 25, SEQ ID No 26, and sequences complementary thereto.

Also provided herein are oligonucleotide probe for detection of a nucleic acid target sequence which is characteristic of the species of the genus *Lactobacillus,* said probe being selected from:
1)-a labelled oligonucleotide which specifically hybridises to *Lactobacillus acidophilus* DNA represented by SEQ ID No 3 or a sequence complementary thereto;
2)-a labelled oligonucleotide which specifically hybridises to *Lactobacillus casei* DNA represented by SEQ ID No 6 or a sequence complementary thereto;
3)-a labelled oligonucleotide which specifically hybridises to *Lactobacillus delbrueckii* DNA represented by SEQ ID No 9 or a sequence complementary thereto;
4)-a labelled oligonucleotide which specifically hybridises to *Lactobacillus fermentum* DNA represented by SEQ ID No 12 or a sequence complementary thereto;
5)-a labelled oligonucleotide which specifically hybridises to *Lactobacillus paracasei* DNA represented by SEQ ID No 15 or a sequence complementary thereto;
6)-a labelled oligonucleotide which specifically hybridises to *Lactobacillus plantarum* DNA represented by SEQ ID No 18 or a sequence complementary thereto;
7)-a labelled oligonucleotide which specifically hybridises to *Lactobacillus reuteri* DNA represented by SEQ ID No 21 or a sequence complementary thereto;
8)-a labelled oligonucleotide which specifically hybridises to *Lactobacillus rhamnosus* DNA represented by SEQ ID No 24 or a sequence complementary thereto;
9)-a labelled oligonucleotide which specifically hybridises to *Lactobacillus* DNA represented by SEQ ID No 27 or a sequence complementary thereto.

Further provided herein is a method of species-specifically detecting species of the genus *Lactobacillus* found in human, particularly human infants, comprising the steps of:
(A) contacting a sample with an oligonucleotide probe in a hybridising solution, wherein said probe is selected from the group consisting of
   1) a labelled oligonucleotide which specifically hybridises to *Lactobacillus acidophilus* DNA represented by SEQ ID No 3 or a sequence complementary thereto;
   2) a labelled oligonucleotide which specifically hybridises to *Lactobacillus casei* DNA represented by SEQ ID No 6 or a sequence complementary thereto;
   3) a labelled oligonucleotide which specifically hybridises to *Lactobacillus delbrueckii* DNA represented by SEQ ID No 9 or a sequence complementary thereto;
   4) a labelled oligonucleotide which specifically hybridises to *Lactobacillus fermentum* DNA represented by SEQ ID No 12 or a sequence complementary thereto;
   5) a labelled oligonucleotide which specifically hybridises to *Lactobacillus paracasei* DNA represented by SEQ ID No 15 or a sequence complementary thereto;
   6) a labelled oligonucleotide which specifically hybridises to *Lactobacillus plantarum* DNA represented by SEQ ID No 18 or a sequence complementary thereto;
   7) a labelled oligonucleotide which specifically hybridises to *Lactobacillus reuteri* DNA represented by SEQ ID No 21 or a sequence complementary thereto;
   8) a labelled oligonucleotide which specifically hybridises to *Lactobacillus rhamnosus* DNA represented by SEQ ID No 24 or a sequence complementary thereto;
   9) a labelled oligonucleotide which specifically hybridises to all *Lactobacillus* DNA represented by SEQ ID No 27 or a sequence complementary thereto, and
(B) determining whether said probe hybridises to nucleic acids in said sample so as to detect whether said species of said genus is present in said sample.

The present invention also encompasses a method of species-specifically detecting species of the genus *Lactobacillus* found in human, particularly human infants, comprising the steps of
a) performing a nucleic acid sequence amplification procedure using a primer set comprising the oligonucleotide primer of SEQ ID No selected from SEQ ID No 1, SEQ ID No 4, SEQ ID No 7, SEQ ID No 10, SEQ ID No 13, SEQ ID No 16, SEQ ID No 19, SEQ ID No 22, SEQ ID No 25, and respectively with the oligonucletide primer of SEQ ID selected from SEQ ID No 2, SEQ ID No 5, SEQ ID No 8, SEQ ID No 11, SEQ ID No 14, SEQ ID No 17, SEQ ID No 20, SEQ ID No 23, SEQ ID No 26; and
b) determining whether the oligonucleotide probe above mentioned hybridises to the nucleic acid target sequence, preferably the oligonucleotide probe is of SEQ ID No selected from SEQ ID No 3, SEQ ID No 6, SEQ ID No 9, SEQ ID No 12, SEQ ID No 15, SEQ ID No 18, SEQ ID No 21, SEQ ID No 24, SEQ ID No 27.

The present method is beneficial for the manufacture of a diagnostic kit. Accordingly, a diagnostic kit is herein provided for the detection in a sample of *Lactobacillus* species selected from *L. acidophilus, L. casei, L. delbrueckii, L. fermentum, L. paracasei, L. plantarum, L. reuteri* and *L. rhamnosus,* by means of hybridisation analysis, comprising at least a DNA probe as mentioned above as well as one or more further means required for hybridisation analysis, such as denaturation liquid, a hybridisation liquid, a washing liquid, a solid carrier, a hybridisation vessel and label detecting means. Also herein provided is a diagnostic kit for the detection in a sample of the above-mentioned *Lactobacillus* species by means of PCR analysis, comprising a set of DNA primers as mentioned above as well as one or more further means required for PCR analysis, such as a polymerase, a polymerisation liquid, an oil overlay, a reaction vessel and means for detecting the amplified DNA. In one embodiment the diagnostic kit comprises at least a DNA probe as described above and a set of primers as described above and optionally further means as mentioned above.

The present probes and methods are particularly suitable for quantifying *Lactobacillus* on a species level in the intestine and/or faeces. The probe and primers with SEQ ID 25, 26, and 27 are not related to specific *Lactobacillus* species but to the genus *Lactobacillus.* SEQ ID 25, 26, 27 are used to quantify the genus *Lactobacillus* and to relate the quantity of specific *Lactobacillus* species to all lactobacilli. Thus it may be said that SEQ ID NOs 1-24 are used for detecting and identifying a species and that SEQ ID NOs 25-27 are used for quantifying this identified species. Preferably *Lactobacillus* species are quantified in infant faeces, since lactobacilli play an important role in the infants' health. The results obtained from such an analysis can be used for selecting suitable *Lactobacillus* species as a probiotic supplement or probiotic food or component in a (helath) nutritional composition and/or selecting non-digestible saccharides selectively stimulating *Lactobacillus* species to be incorporated into a (health) nutritional composition. These probiotics and/or prebiotics are preferably to be administered to the subject low in specific *Lactobacillus* species in order to rebalance the intestinal flora on a *Lactobacillus* species level and prevent or treat disorders associated with this imbalance. Preferably, the disorder is selected from the group consisting of allergy, atopic disease, eczema, diarrhoea, intestinal inflammation and infections. Likewise, the present probes and methods are preferably applied for selecting probiotics and/or non-digestible saccharides for administration to animals in order to promote animal health, more preferably the gastro-intestinal health of animals. Thus a method for preparing a nutritional composition is provided, said method comprising selecting suitable *Lactobacillus* species and/or selecting non-digestible saccharides selectively stimulating *Lactobacillus* species by applying the oligonucleotide probes according to the present invention and preferably the oligonucleotide primers according to the present invention, preferably in a method according to the present invention and incorporating said selected *Lactobacillus* species and/or non-digestible saccharides into a (health) nutritional composition.

The present probes and methods are preferably used for quantification and species determination of systemic infections by species from the genus *Lactobacillus.*

The present probes and methods are preferably used in controlling and/or monitoring food fermentation by *Lactobacillus* species.

### Example:

### Validation and optimisation of the developed probes and primers for Lactobacilli

The bacterial strains used to validate the assays for the relative quantification of the different *Lactobacillus* species are listed in Table 2.

**Table 2: Bacterial strains (origin) used in this study.**

| ***Lactobacillus* Strains** | **Origin**^{**a**} | **Other Strains** | **Origin**^{**a**} |
|---|---|---|---|
| *L. acidophilus* | ATCC 4356 | *Bacillus cereus* | ATCC 11778 |
| | ATCC 521 | *Bacteroides fragilis* | LMG 10263^{T} |
| | DSM 20079^{T} | *Brevibacterium casei* | ATCC 35513^{T} |
| *L. amylovorus* | DSM 20552 | *Clostridium difficile* | ATCC 9689^{T} |
| *L. bavaricus* | JCM 1128 | *Enterococcus feacalis* | DSM 20478^{T} |
| *L. brevis* | LMG 18022 | *Escherichia coli* | ATCC 35218 |
| *L. bulgaricus* | ATCC 11842^{T} | *Listeria monocytogenes* | ATCC 7644 |
| *L. casei* | ATCC 393^{T} | *Pediococcus acidilactici* | DSM 20284^{T} |
| | DSM 20011^{T} | *Propionibacterium avidum* | DSM 4901 |
| *L. crispatus* | ATCC 33820^{T} | *Pseudomonas aeruginosa* | DSM 1117 |
| *L. curvatus* | ATCC 51436 | *Saccharomyces cerevisiae* | DSM 2548 |
| *L. delbrueckii* | JCM 1106 | *Salmonella typhimurium* | |
| | JCM 1248^{T} | *Staphylococcus aureus* | ATCC 14028 |
| | | | ATCC 29213 |
| *L. fermentum* | DSM 20052^{T} | *Bifidobacterium* | ATCC 15703^{T} |
| *L. gasseri* | LMG 11496^{T} | *adolescentis* | |
| | | *Bifidobacterium angulatum* | DSM 20098^{T} |
| | | *Bifidobacterium bifidum* | DSM 20456^{T} |
| *L. helveticus* | CNRZ 32 | *Bifidobacterium animalis* | ATCC 25527^{T} |
| *L. johnsonii* | ATCC 33200^{T} | *Bifidobacterium gallicum* | DSM 20093^{T} |
| *L. kefir* | LMG 11496 | *Bifidobacterium dentium* | ATCC 27534^{T} |
| *L. kefirgranum* | DSM 10550^{T} | *Bifidobacterium breve* | ATCC 15700^{T} |
| *L. paracasei* | ATCC 11582 | *Bifidobacterium* | ATCC 27539^{T} |
| | | *catenulatum* | |
| | ATCC 27216 | *Bifidobacterium infantis* | LMG 8811^{T} |
| *L. pentosus* | JCM 8334 | *Bifidobacterium longum* | ATCC 15707^{T} |
| | JCM 8338 | | |
| *L. plantarum* | DSM 20174^{T} | | |
| | NCIMB 8826 | | |
| *L. reuteri* | LMG 9213^{T} | | |
| *L. rhamnosus* | ATCC 53103 | | |
| | ATCC 7469^{T} | | |
| *L. sake* | DSM 6333 | | |
| *L. salivarius* | ATCC 11741^{T} | | |

| | | | |
|---|---|---|---|
| ^{a} ATCC: American Type Culture Collection; CNRZ: Centre National de Recerches Zootechniques, France; | | | |
| DSM: Deutsche Sammlung von Mikroorganismen und Zellkulturen, Germany; JCM: Japan Collection of Microorganisms, Japan; LMG: Laboratory for Microbiology, University of Gent, Belgium; NCIMB: National Collections of Industrial and Marine Bacteria, UK; ^{T}Type strain | | | |

All strains of lactobacilli, bifidobacteria, propionibacteria, enterococci, pediococci and *Saccharomyces* were cultured overnight under suitable conditions stored at -20°C. DNA was extracted by thawing 5 ml of frozen overnight cultures by methods known in the art. and stored at -20°C.

The specificity of each duplex 5' nuclease assay was tested by performing a 25 µl amplification of the different strains. These 25 µl PCR reactions were performed using 2.5 µl DNA template, 12.5 µl TaqMan Universal Master Mix (Applied Biosystems), optimised concentrations of the primers and probes (see table 3), followed by running the TaqMan Universal Temperature Profile, on the ABI Prism 7700 (Applied Biosystems, Nieuwerkerk a/d IJssel, NL). All of the 5' nuclease assays were specific for the *Lactobacillus* species for which they were developed and the 5'nuclease assay for determination of the genus *Lactobacillus* detected all *Lactobacillus* species tested, but no other bacteria like *Propionibacterium, Enterococcus* or *Pediococcus* species.

The optimised concentrations of the primers and probes are given in table 3. The CV values for reproducibility and repeatability for the different kind of 5' nuclease assays were determined and can be found in table 4.

**Table 3: Optimised final primer and probe concentrations used in the different duplex 5' nuclease assays**

| Target | 5' nuclease assay | Forward Primer (nM) | Reverse Primer (nM) | Probe (nM) |
|---|---|---|---|---|
| *L. acidophilus* | *L. acidophilus* | 900 | 900 | 200 |
| | Genus *Lactobacillus* | 900 | 900 | 200 |
| *L. casei* | *L. casei* | 900 | 900 | 200 |
| | Genus *Lactobacillus* | 300 | 300 | 50 |
| *L. delbrueckii* | *L. delbrueckii* | 300 | 300 | 100 |
| | Genus *Lactobacillus* | 900 | 900 | 200 |
| *L. fermentum* | *L. fermentum* | 300 | 300 | 100 |
| | Genus *Lactobacillus* | 300 | 300 | 100 |
| *L. paracasei* | *L. paracasei* | 300 | 300 | 100 |
| | Genus *Lactobacillus* | 300 | 300 | 100 |
| *L. plantarum* | *L. plantarum* | 300 | 300 | 100 |
| | Genus *Lactobacillus* | 300 | 300 | 100 |
| *L. reuteri* | *L. reuteri* | 300 | 300 | 100 |
| | Genus *Lactobacillus* | 900 | 900 | 200 |
| *L. rhamnosus* | *L. rhamnosus* | 900 | 450 | 200 |
| | Genus *Lactobacillus* | 150 | 100 | 100 |
| Genus *Lactobacillus* | Genus *Lactobacillus* | 600 | 600 | 100 |
| | All bacteria | 300 | 300 | 100 |

**Table 4: The CV values for reproducibility and repeatability of the 5' nuclease assays**

| Target | Reproducibility^{a} (CV) | Repeatability^{b} (CV) |
|---|---|---|
| *L. acidophilus* | 0.109 | 0.127 |
| *L. casei* | 0.058 | 0.063 |
| *L. delbrueckii* | 0.059 | 0.035 |
| *L. fermentum* | 0.067 | 0.048 |
| *L. paracasei* | 0.052 | 0.110 |
| *L. plantarum* | 0.134 | 0.135 |
| *L. reuteri* | 0.062 | 0.113 |
| *L. rhamnosus* | 0.047 | 0.053 |

| | | |
|---|---|---|
| ^{a} reproducibility is determined by testing monocultures (100%) in ten fold and calculation of the CV (%) based on the gained results | | |
| ^{b} repeatability is determined by testing monocultures (100%) three times in four fold and calculation of the CV (%) based on results gained. | | |

The developed and validated duplex 5' nuclease assays show a good specificity, sensitivity, reproducibility and repeatability and can therefore be seen as a good tool for enumeration of the different *Lactobacillus* species in faeces or intestinal biopsies.

## Claims

1. Oligonucleotides comprising SEQ ID selected from SEQ ID No 1, SEQ ID No 2, SEQ ID No 4, SEQ ID No 5, SEQ ID No 7, SEQ ID No 8, SEQ ID No 10, SEQ ID No 11, SEQ ID No 13, SEQ ID No 14, SEQ ID No 16, SEQ ID No 17, SEQ ID No 19, SEQ ID No 20, SEQ ID No 22, SEQ ID No 23, SEQ ID No 25, SEQ ID No 26, and sequences complementary thereto.

2. Oligonucleotide probe for detection of a nucleic acid target sequence which is characteristic of the species of the genus *Lactobacillus,* said probe being selected from:
1) a labelled oligonucleotide which specifically hybridises to *L. acidophilus* DNA represented by SEQ ID No 3 or a sequence complementary thereto;
2) a labelled oligonucleotide which specifically hybridises to *L. casei* DNA represented by SEQ ID No 6 or a sequence complementary thereto;
3) a labelled oligonucleotide which specifically hybridises to *L. delbrueckii* DNA represented by SEQ ID No 9 or a sequence complementary thereto;
4) a labelled oligonucleotide which specifically hybridises to *L. fermentum* DNA represented by SEQ ID No 12 or a sequence complementary thereto;
5) a labelled oligonucleotide which specifically hybridises to *L. paracasei* DNA represented by SEQ ID No 15 or a sequence complementary thereto;
6) a labelled oligonucleotide which specifically hybridises to *L. plantarum* DNA represented by SEQ ID No 18 or a sequence complementary thereto;
7) a labelled oligonucleotide which specifically hybridises to *L. reuteri* DNA represented by SEQ ID No 21 or a sequence complementary thereto;
8) a labelled oligonucleotide which specifically hybridises to *L. rhamnosus* DNA represented by SEQ ID No 24 or a sequence complementary thereto;
9) a labelled oligonucleotide which specifically hybridises to the genus *Lactobacillus* DNA represented by SEQ ID No 27 or a sequence complementary thereto.

3. A method of species-specifically detecting species of the genus *Lactobacillus* found in human, particularly human infants, comprising the steps of:
(A) contacting a sample with an oligonucleotide probe in a hybridising solution, wherein said probe is selected from the group consisting of
1) a labelled oligonucleotide which specifically hybridises to *L. acidophilus* DNA represented by SEQ ID No 3 or a sequence complementary thereto;
2) a labelled oligonucleotide which specifically hybridises to *L. casei* DNA represented by SEQ ID No 6 or a sequence complementary thereto;
3) a labelled oligonucleotide which specifically hybridises to *L. delbrueckii* DNA represented by SEQ ID No 9 or a sequence complementary thereto;
4) a labelled oligonucleotide which specifically hybridises to *L. fermentum* DNA represented by SEQ ID No 12 or a sequence complementary thereto;
5) a labelled oligonucleotide which specifically hybridises to *L. paracasei* DNA represented by SEQ ID No 15 or a sequence complementary thereto;
6) a labelled oligonucleotide which specifically hybridises to *L. plantarum* DNA represented by SEQ ID No 18 or a sequence complementary thereto;
7) a labelled oligonucleotide which specifically hybridises to *L. reuteri* DNA represented by SEQ ID No 21 or a sequence complementary thereto;
8) a labelled oligonucleotide which specifically hybridises to *L. rhamnosus* DNA represented by SEQ ID No 24 or a sequence complementary thereto;
9) a labelled oligonucleotide which specifically hybridises to the genus *Lactobacillus* DNA represented by SEQ ID No 27 or a sequence complementary thereto, and
(B) determining whether said probe hybridises to nucleic acids in said sample so as to detect whether said species of said genus is present in said sample.

4. A method of species-specifically quantifying the detected species of the genus *Lactobacillus* found in humans, particularly in human faeces, according to claim 3, wherein said quantifying method is real time PCR.

5. A method of species-specifically detecting species of the genus *Lactobacillus* found in human, particularly human infants, comprising the steps of:
a) performing a nucleic acid sequence amplification procedure using a primer set comprising the oligonucleotide primer of claim 1, preferably the oligonucleotide primer of SEQ ID No selected from SEQ ID No 1, SEQ ID No 4, SEQ ID No 7, SEQ ID No 10, SEQ ID No 13, SEQ ID No 16, SEQ ID No 19, SEQ ID No 22, SEQ ID No 25, and respectively with the oligonucleotide primer of SEQ ID selected from SEQ ID No 2, SEQ ID No 5, SEQ ID No 8, SEQ ID No 11, SEQ ID No 14, SEQ ID No 17, SEQ ID No 20, SEQ ID No 23, SEQ ID No 26; and
b) determining whether an oligonucleotide probe of claim 2, preferably respectively the oligonucleotide probe of SEQ ID No selected from SEQ ID No 3, SEQ ID No 6, SEQ ID No 9, SEQ ID No 12, SEQ ID No 15, SEQ ID No 18, SEQ ID No 21, SEQ ID No 24, SEQ ID No 27, hybridises to a nucleic acid target sequence.

6. A diagnostic kit for the detection in a sample of *Lactobacillus* species selected from *L. acidophilus, L. casei, L. delbrueckii, L. fermentum, L. paracasei. L. plantarum, L. reuteri,* and *L. rhamnosus,* by means of hybridisation analysis, comprising at least a DNA probe according to claim 2 as well as one or more further means required for hybridisation analysis, such as denaturation liquid, a hybridisation liquid, a washing liquid, a solid carrier, a hybridisation vessel and label detecting means.

7. A diagnostic kit for the detection in a sample of *Lactobacillus* species selected from *L. acodophilus, L. casei, L. delbrueckii, L. fermentum, L.paracasei, L. plantarum, L. reuteri,* and *L. rhamnosus* by means of PCR analysis, comprising a set of DNA primers according to claim 1 as well as one or more further means required for PCR analysis, such as a polymerase, a polymerisation liquid, an oil overlay, a reaction vessel and means for detecting the amplified DNA.

8. Use of the oligonucleotide sequences and methods of any of the preceding claims for quantifying *Lactobacillus* species in faeces.

9. Use of the oligonucleotide sequences and methods of any of the preceding claims for selecting *Lactobacillus* species and/or non-digestible saccharides selectively stimulating the growth of *Lactobacillus* species, to be incorporated into a nutritional composition.


SEQUENCE LISTING

<110>  N.V. Nutricia

<120>  Lactobacillus specific probes

<130>  P6001754EP1US

<160>  27

<170>  PatentIn version 3.3

<210>  1
<211>  23
<212>  DNA
<213>  Artificial

<220>
<223>  primer

<400>  1
gaaagagccc aaaccaagtg att                                              23


<210>  2
<211>  27
<212>  DNA
<213>  Artificial

<220>
<223>  primer

<400>  2
cttcccagat aattcaacta tcgctta                                          27


<210>  3
<211>  20
<212>  DNA
<213>  Artificial

<220>
<223>  probe

<400>  3
taccactttg cagtcctaca                                                  20


<210>  4
<211>  28
<212>  DNA
<213>  Artificial

<220>
<223>  primer

<400>  4
ctataagtaa gctttgatcc ggagattt                                         28


<210>  5
<211>  22
<212>  DNA
<213>  Artificial

<220>
<223>  primer

<400>  5
cttcctgcgg gtactgagat gt                                               22


<210>  6
<211>  21

<212>  DNA
<213>  Artificial

<220>
<223>  probe

<400>  6
acaagctatg aattcacttg c                                                21


<210>  7
<211>  30
<212>  DNA
<213>  Artificial

<220>
<223>  primer

<400>  7
cacttgtacg ttgaaaactg aatatcttaa                                       30


<210>  8
<211>  20
<212>  DNA
<213>  Artificial

<220>
<223>  primer

<400>  8
cgaactctct cggtcgcttt                                                  20


<210>  9
<211>  18
<212>  DNA
<213>  Artificial

<220>
<223>  probe

<400>  9
ccgagaatca ttgagatc                                                    18


<210>  10
<211>  20
<212>  DNA
<213>  Artificial

<220>
<223>  primer

<400>  10
aaccgagaac accgcgttat                                                  20


<210>  11
<211>  29
<212>  DNA
<213>  Artificial

<220>
<223>  primer

<400>  11
acttaacctt actgatcgta gatcagtca                                        29


<210>  12
<211>  19
<212>  DNA
<213>  Artificial

<220>
<223>  probe

<400>  12
taatcgcata ctcaactaa                                                    19


<210>  13
<211>  29
<212>  DNA
<213>  Artificial

<220>
<223>  primer

<400>  13
acatcagtgt attgcttgtc agtgaatac                                         29


<210>  14
<211>  22
<212>  DNA
<213>  Artificial

<220>
<223>  primer

<400>  14
cctgcgggta ctgagatgtt tc                                                22


<210>  15
<211>  13
<212>  DNA
<213>  Artificial

<220>
<223>  probe

<400>  15
tgccgccggc cag                                                          13


<210>  16
<211>  24
<212>  DNA
<213>  Artificial

<220>
<223>  primer

<400>  16
tggatcacct cctttctaag gaat                                              24


<210>  17
<211>  27
<212>  DNA
<213>  Artificial

<220>
<223>  primer

<400>  17
tgttctcggt ttcattatga aaaaata                                           27


<210>  18
<211>  19
<212>  DNA
<213>  Artificial

<220>

<223>  probe

<400>  18
acattcttcg aaactttgt                                                    19


<210>  19
<211>  21
<212>  DNA
<213>  Artificial

<220>
<223>  primer

<400>  19
accgagaaca ccgcgttatt t                                                 21


<210>  20
<211>  32
<212>  DNA
<213>  Artificial

<220>
<223>  primer

<400>  20
cataacttaa cctaaacaat caaagattgt ct                                     32


<210>  21
<211>  18
<212>  DNA
<213>  Artificial

<220>
<223>  probe

<400>  21
atcgctaact caattaat                                                     18


<210>  22
<211>  19
<212>  DNA
<213>  Artificial

<220>
<223>  primer

<400>  22
cggctggatc acctccttt                                                    19


<210>  23
<211>  21
<212>  DNA
<213>  Artificial

<220>
<223>  primer

<400>  23
gcttgagggt aatcccctca a                                                 21


<210>  24
<211>  15
<212>  DNA
<213>  Artificial

<220>
<223>  probe

<400>  24
cctgcacaca cgaaa                                                        15


<210>  25
<211>  20
<212>  DNA
<213>  Artificial

<220>
<223>  primer

<400>  25
tggatgcctt ggcactagga                                                   20


<210>  26
<211>  26
<212>  DNA
<213>  Artificial

<220>
<223>  primer

<400>  26
aaatctccgg atcaaagctt acttat                                            26


<210>  27
<211>  20
<212>  DNA
<213>  Artificial

<220>
<223>  probe

<400>  27
tattagttcc gtccttcatc                                                   20